# EUROPÄISCHE PATENTSCHRIFT

(11) **EP 2 143 468 B1**
(45) Veröffentlichungstag und Bekanntmachung des Hinweises auf die Patenterteilung: **04.03.2015**
(21) Anmeldenummer: 09160085.8
(22) Anmeldetag: 13.05.2009
(51) Int. Cl.: A61N 1/372, G06F 19/00, H04B 1/16

(54) **Implantierbares medizinisches Gerät**
Implantable medical device
Appareil médical implantable

(30) Priorität: 11.06.2008 DE 102008002369
(43) Veröffentlichungstag der Anmeldung: 13.01.2010
(73) Patentinhaber: Biotronik CRM Patent AG, 6341 Baar (CH)
(72) Erfinder: Dörr, Thomas, 12437, Berlin (DE)
(74) Vertreter: Lindner-Vogt, Karin L.

(56) Entgegenhaltungen:
- EP-A- 1 892 011
- WO-A-03/024322
- WO-A-2006/114297
- US-A1- 2003 149 459
- US-B1- 6 472 991

## Beschreibung

Die Erfindung betrifft ein implantierbares medizinisches Gerät, wie beispielsweise implantierbare Herzschrittmacher oder implantierbare Cardioverter/Defibrillatoren (ICD). Die Erfindung ist jedoch nicht auf diese speziellen implantierbaren medizinischen Geräte beschränkt, sondern betrifft grundsätzlich alle elektronisch gesteuerten implantierbaren medizinischen Geräte.

Solche implantierbaren medizinischen Geräte, wie Herzschrittmacher oder ICDs, sind heute häufig mit einer bidirektionalen Funkschnittstelle ausgestattet, die es erlaubt, diese medizinischen Geräte mittels eines externen Gerätes zu programmieren oder Daten aus den medizinischen Geräten, wie beispielsweise von dem medizinischen Gerät erfasste physiologische Daten, abzufragen. Aufgrund der Tatsache, dass implantierbare medizinische Geräte ein möglichst kleines Volumen aufweisen sollen und daher notwendigerweise für Ihren Betrieb meist nur eine begrenzte Energiemenge, die in der Regel durch eine Batterie bereitgestellt ist, zur Verfügung haben, ist es üblich, dass die jeweilige bidirektionale Funkstellenschnittstelle nicht permanent in Betrieb ist, sondern möglichst nur dann aktiviert wird, wenn sie wirklich gebraucht wird. Auf diese Weise kann der Gesamtenergieverbrauch der bidirektionalen Funkschnittstelle über einen längeren Zeitraum in Grenzen gehalten werden. Die bidirektionale Funkschnittstelle kann somit zwischen einem Abschaltzustand und einem Einschaltzustand hin und her wechseln, wobei die bidirektionale Funkschnittstelle im Abschaltzustand fast keine oder nur sehr wenig Energie verbraucht und nur im Einschaltzustand in der Lage ist, Daten drahtlos zu empfangen und zu senden.

Aus diesem Grunde benötigten derzeitige medizinische Geräte zur Programmierung über eine bidirektionale Funkschnittstelle einen Aktivierungsmechanismus, um die bidirektionale Funkschnittstelle des medizinischen Gerätes einzuschalten. Zur Aktivierung der bidirektionalen Funkschnittstelle, d.h. um die bidirektionale Funkschnittstelle zu einem Wechsel von dem Abschaltzustand in den Einschaltzustand zu veranlassen, sind heute üblicherweise weise Aktivatoren in Form externer separater Geräte oder der Programmierkopf für die induktive Telemetrie eines üblichen Programmiergerätes vorgesehen. Absehbar ist auch, dass zukünftig zusätzliche stromarme Empfänger in einem anderen Frequenzbereich als dem der bidirektionalen Funkschnittstelle zur Aktivierung der bidirektionalen Funkschnittstelle vorgesehen sind. Beispiele die Aktivierung der bidirektionalen Funkschnittstelle mittels induktiver Telemetrie sind in US2003/149459A1, WO03/024322A und WO2006/114297A offenbart.

Nachteile der genannten Lösungen sind die eines zusätzlichen externen Gerätes, welches mit Kosten verbunden ist, Probleme, die das Einbringen des externen Gerätes in den sterilen Bereich in einem Operationssaal mit sich bringt oder das Risiko des Verlustes des externen Gerätes mit sich bringt, oder aber die Nachteile eines zusätzlichen Empfängers im implantierbaren medizinischen Gerät. Letzterer verursacht Kosten, verbraucht Strom und muss zugelassen werden. Außerdem ist die Antennenabstimmung bei der Verwendung einer zweiten Hochfrequenz kritisch, da hier ein Kompromiss zwischen den Frequenzen des zusätzlichen Empfängers zur Aktivierung der bidirektionalen Funkschnittstelle und den Frequenzen für die bidirektionale Funkschnittstelle gemacht werden muss oder aber eine zweite Antenne im elektronischen Implantat zu integrieren ist.

Der Erfindung liegt, ausgehend von diesem Stand der Technik, die Aufgabe zugrunde, ein implantierbares medizinisches Gerät mit einer Funkschnittstelle zu schaffen, welches einen möglichst einfachen und kostengünstigen Mechanismus zur Fernaktivierung der Funkschnittstelle aufweist.

Erfindungsgemäß wird diese Aufgabe durch ein implantierbares medizinisches Gerät, insbesondere durch einen implantierbaren Herzschrittmacher oder einen implantierbaren Cardioverter/Defibrillator (ICD) gelöst, der eine Steuereinheit aufweist, die mit einer bidirektionalen Funkschnittstelle für eine drahtlose Datenübertragung mittels eines elektrischen Wechselfeldes und einer magnetischen Schnittstelle für den Empfang von Daten über ein magnetisches Wechselfeld verbunden ist. Dabei kann die bidirektionale Funkschnittstelle wenigstens einen Abschaltzustand und einen Einschaltzustand einnehmen, wobei eine drahtlose Datenübertragung nur im Einschaltzustand möglich ist und die Funkschnittstelle im Abschaltzustand keine oder fast keine Energie benötigt. Die magnetische Schnittstelle ist ausgebildet, mittels eines magnetischen Wechselfeldes zu übertragende Steuersignale seitens eines externen Gerätes permanent empfangen zu können. Erfindungsgemäß ist die magnetische Schnittstelle weiter ausgebildet, ein über ein magnetisches Wechselfeld übertragenes Datenübertragungs-Startsignal zu empfangen und derart zu verarbeiten, beispielsweise zu decodieren, dass die magnetische Schnittstelle oder die Steuereinheit in Reaktion auf den Empfang eines solchen Datenübertragungs-Startsignals ein Funkschnittstellen-Einschaltsignal erzeugt. Die bidirektionale Funkschnittstelle ist ihrerseits erfindungsgemäß wenigstens mittelbar mit der magnetischen Schnittstelle verbunden - beispielsweise über die Steuereinheit - und ausgebildet, auf ein Funkschnittstellen-Einschaltsignal hin von dem Abschaltzustand in den Einschaltzustand zu wechseln.

Die Erfindung beruht auf der Erkenntnis, dass in typischen implantierbaren medizinischen Geräten auch eine magnetische Schnittstelle für die induktive Telemetrie vorhanden ist, die zur Aktivierung der bidirektionalen Funkschnittstelle genutzt werden kann. Derzeit befindet sich nämlich in gängigen elektronischen Implantaten eine Sende- und Empfangsantenne für die induktive Telemetrie im Inneren eines hermetisch geschlossen Metallgehäuses, das nur durch ein magnetisches Wechselfeld durchdrungen werden kann, nicht jedoch durch ein elektrisches Wechselfeld. Daher werden bekannte Kommunikationsverfahren in diesem Zusammenhang als induktive Verfahren beschrieben. Solche bekannten Kommunikationsverfahren sind die Kommunikation zwischen einem Programmiergerät und einem Implantat mittels eines magnetischen Wechselfeldes. Im Unterschied zu den derzeit bekannten Aktivierungsmechanismen mittels induktiver Telemetrie (Programmierkopf oder externer Aktivator) wird die induktive Telemetrie erfindungsgemäß nicht bidirektional sondern nur unidirektional angewendet. D.h. es wird das Kommando (Datenübertragungsstartsignal) zu Aktivierung der Funkschnittstelle mittels induktiver Telemetrie (magnetisches Wechselfeld) an das elektronische Implantat übertragen, die Rückantwort erfolgt aber bereits mittels der Hochfrequenz-Funktelemetrie. Der Vorteil besteht darin, dass die Sendeleistung des externen Gerätes für die Übertragung des Datenübertragungsstartsignals an das Implantat fast unbegrenzt gesteigert werden kann und die mögliche Geometrie der Sendespule weitgehend optimiert werden kann. Die Optimierung der induktiven Sendespule und Energie im elektronischen Implantat ist dagegen aufgrund der kleinen Geometrie und der Limitation der Stromversorgung stark eingeschränkt.

Das Datenübertragungs-Startsignal ist dabei durch eine spezifische Sequenz im magnetischen Wechselfeld codiert, so dass es von der magnetischen Schnittstelle (im Folgenden auch als induktive Telemetrieeinheit bezeichnet) eindeutig als Datenübertragungs-Startsignal decodiert und identifiziert werden kann.

Ein weiterer Aspekt der Erfindung besteht in einem zum implantierbaren medizinischen Gerät komplementären externen Gerät. Dieses externe Gerät besitzt ebenfalls eine bidirektionale Funkschnittstelle für die Übertragung von Daten mittels eines hochfrequenten elektromagnetischen Wechselfeldes sowie eine induktive Sendeeinheit zum Übertragen eines Datenübertragungs-Startsignals mittels eines magnetischen Wechselfeldes.

Ein erfindungsgemäßes Gesamtsystem umfasst ein erfindungsgemäßes implantierbares medizinisches Gerät und ein erfindungsgemäßes externes Gerät. Um eine bidirektionale Datenübertragung zwischen dem externen Gerät und dem implantierbaren medizinischen Gerät auszulösen, sendet das externe Gerät zunächst über die induktive Sendeeinheit ein Datenübertragungs-Startsignal. Falls dieses Datenübertragungs-Startsignal von einem implantierbaren medizinischen Gerät über dessen magnetische Schnittstelle empfangen wird, wird das Datenübertragungs-Startsignal seitens der magnetischen Schnittstelle oder der Steuereinheit des implantierbaren medizinischen Gerätes decodiert und ein Funkschnittstellen-Einschaltsignal erzeugt, welches bewirkt, dass die Funkschnittstelle des implantierbaren medizinischen Gerätes eingeschaltet wird.

Erfindungsgemäß ist das implantierbare medizinische Gerät so ausgebildet, dass es nach Einschalten der Funkschnittstelle infolge des Empfangs eines Datenübertragungs-Startsignals und des daraufhin generierten Funkschnittstellen-Einschaltsignals den Empfang eines Datenpaketes über die Funkschnittstelle abwartet, bevor das implantierbare medizinische Gerät über die Funkschnittstelle selbst ein Datenpaket aussendet. In diesem Zusammenhang ist das externe Gerät dazu ausgebildet, nach dem Aussenden eines Datenübertragungs-Startsignals über die induktive Sendeeinheit ein initiales Datenpaket über die Funkschnittstelle des externen Gerätes auszusenden. Erreicht das vom externen Datengerät ausgesendete Datenübertragungs-Startsignal das implantierbare medizinische Gerät und bewirkt das Einschalten von dessen Funkschnittstelle und erreicht daraufhin das initiale Datenpaket, welches von dem externen Gerät ausgesendet wurde, die Funkschnittstelle des implantierbaren medizinischen Gerätes, wird die bidirektionale Datenübertragung zwischen externem Gerät und implantierbarem medizinischen Gerät gesta rtet.

Gemäß bevorzugter Ausführungsvariante ist das Datenübertragungs-Startsignal für ein jeweiliges implantierbares medizinisches Gerät spezifisch. In Bezug auf ein bevorzugtes erfindungsgemäßes implantierbares medizinisches Gerät bedeutet dies, dass dessen magnetische Schnittstelle oder dessen Steuereinheit dazu ausgebildet ist, nach Empfang eines Datenübertragungs-Startsignals nur dann ein Funkschnittstellen-Einschaltsignal zu erzeugen, wenn das über die magnetische Schnittstelle empfangene Datenübertragungs-Startsignal eine für das spezielle implantierbare medizinische Gerät spezifische Geräte-Kennung enthält.

Das implantierbare medizinische Gerät bzw. dessen Steuereinheit oder dessen magnetische Schnittstelle ist dann dazu ausgebildet, nur auf Datenübertragungs-Startsignale mit einer spezifischen Kennung anzusprechen.

Gemäß der Erfindung ist das externe Gerät dazu ausgebildet, ein Datenübertragungs-Startsignal zu generieren, welches eine Funkkanalkennung enthält, welche einen für die bidirektionale Datenübertragung über die Funkschnittstelle zu verwendenden Übertragungskanal (Frequenzbereichs) identifiziert. Entsprechend ist das implantierbare medizinische Gerät dazu ausgebildet, ein entsprechendes Datenübertragungs-Startsignal über die magnetische Schnittstelle zu empfangen und derart zu verarbeiten, dass die Funkkanalkennung decodiert wird und anschließend die Funkschnittstelle zur Datenübertragung in diesem Übertragungskanalkanal eingerichtet wird.

Gemäß der Erfindung ist das externe Gerät dazu ausgebildet, vor dem Generieren eines Datenübertragungs-Startsignals, welches eine Funkkanalkennung eines zu verwendenden Übertragungskanals beinhaltet, zunächst die zur Verfügung stehenden Übertragungskanäle zu überprüfen (zu scannen), um einen möglichst freien und möglichst ungestörten Übertragungskanal zu ermitteln. In diesem Zusammenhang ist das externe Gerät weiter dazu ausgebildet, dem auszusendenden Datenübertragungs-Startsignal die Funkkanalkennung desjenigen Übertragungskanals hinzuzufügen, der frei und am wenigsten gestört ist.

Besonders bevorzugt ist ein System und sind Systemkomponenten (implantierbares medizinisches Gerät und externes Gerät), bei denen das Datenübertragungs-Startsignal sowohl eine für ein jeweiliges implantierbares medizinisches Gerät spezifische Gerätekennung als auch eine Funkkanalkennung im zuvor beschriebenen Sinne enthält.

Darüber hinaus kann das implantierbare medizinische Gerät so ausgestaltet sein, dass die Aktivierbarkeit der Funkschnittstelle mittels eines an der magnetischen Schnittstelle generierten Funkschnittstellen-Einschaltsignals zu aktivieren bzw. zu deaktivieren ist. Dieses Aktivieren bzw. Deaktivieren kann durch entsprechende Steuerkommandos erfolgen, die über die magnetische Schnittstelle oder die Funkschnittstelle oder über beide zu empfangen sind.

Auf diese Weise kann das Einschalten der Funkschnittstelle über ein entsprechendes Datenübertragungs-Startsignal gezielt deaktiviert werden. Dies ist insbesondere dann hilfreich, wenn das Datenübertragungs-Startsignal nicht gerätespezifisch ist, also keine für ein jeweiliges implantierbares medizinisches Gerät spezifische Gerätekennung enthält. So ist es möglich, bei solchen implantierbaren medizinischen Geräten das Einschalten der Funkschnittstelle via Datenübertragungs-Startsignal und entsprechendes Funkschnittstellen-Einschaltsignal zu deaktivieren, bei denen in absehbarer Zeit keine bidirektionale Datenübertragung ansteht. Entsprechend wird die Möglichkeit des Einschaltens der Funkschnittstelle via per magnetisches Wechselfeld übertragenen Datenübertragungs-Startsignalen nur bei denjenigen implantierbaren medizinischen Geräten aktiviert, bei denen kurzfristig eine bidirektionale Datenübertragung über die Funkschnittstelle gewünscht ist. So kann verhindert werden, dass die Anzahl der aktivierten implantierbaren medizinischen Geräte die Anzahl der zur Verfügung stehenden Funk-Übertragungskanäle überschreitet.

Im Zusammenhang mit einem Datenübertragungs-Startsignal, das von mehreren implantierbaren medizinischen Geräten gleichzeitig empfangen werden kann, ist es vorteilhaft, wenn das externe Gerät dem Datenübertragungs-Startsignal für über eine entsprechende Gerätekennung identifizierte implantierbare medizinische Geräte jeweils Antwortzeiten zuordnet. Die implantierbaren medizinischen Geräte sind in diesem Zusammenhang dazu ausgebildet, dass sie eine Datenübertragung über die Funkschnittstelle nur zu den jeweils zugewiesenen Antwortzeiten ermöglichen. Auf diese Weise können Kollisionen bei der Datenübertragung über die bidirektionalen Funkschnittstellen vermieden werden.

Die eingangs genante Aufgabe wird erfindungsgemäß auch durch ein Verfahren zum initiieren einer bidirektionalen Datenübertragung zwischen einem implantierbaren medizinischen Gerät mit einer magnetischen Schnittstelle und einer Funkschnittstelle und einem externen Gerät mit einer induktiven Sendeeinheit und einer Funkschnittstelle gelöst, bei dem zunächst eine Datenübertragungsstartsignal von der der induktiven Sendeeinheit zur magnetischen Schnittstelle übertragen wird und daraufhin die Funkschnittstelle des implantierbaren medizinischen Gerätes in ihren Einschaltzustand versetzt wird.

Weitere vorteilhafte Verfahrensvarianten ergeben sich aus den bevorzugten Vorrichtungsvarianten sowie der nachfolgenden Beschreibung von Ausführungsbeispielen.

Die Erfindung soll nun anhand von Ausführungsbeispielen in Bezug auf die Figuren näher erläutert werden. Von diesen zeigt:
- Fig. 1:: Ein implantierbares medizinisches Gerät und ein externes Gerät gemäß einer ersten Ausführungsvariante der Erfindung;
- Fig. 2:: ein alternatives externes Gerät;
- Fig. 3:: eine erste Variante des Ablaufs der Aktivierung einer bidirektionalen Datenkommunikation; und
- Fig. 4:: einen Ablauf einer alternativen Indizierung einer bidirektionalen Datenkommunikation zwischen implantierbarem medizinischen Gerät und externem Gerät.

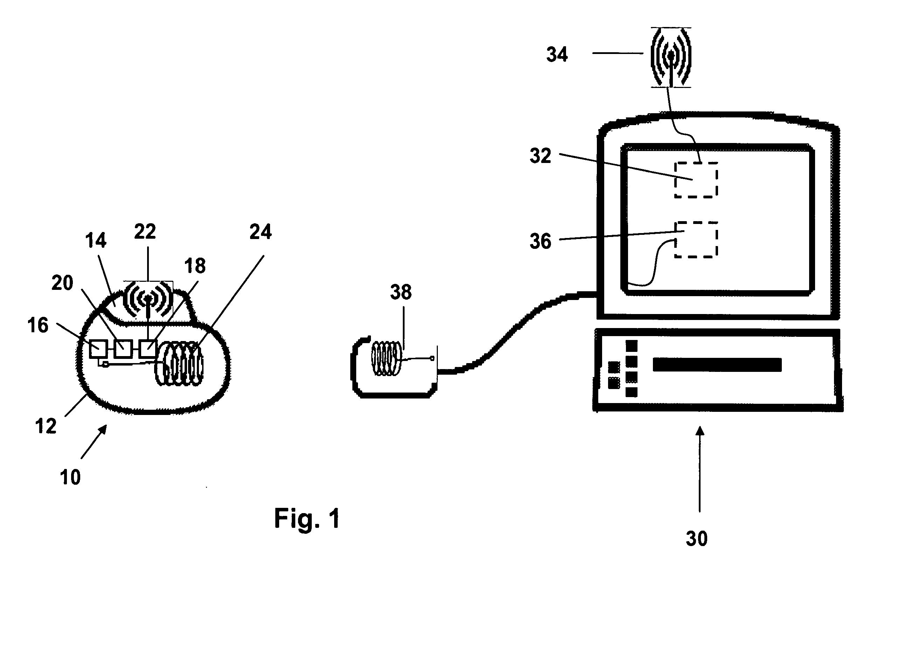

Fig. 1 zeigt ein implantierbares medizinisches Gerät 10 in Verbindung mit einem externen Gerät 30, die zusammen ein System zur bidirektionalen Datenübertragung zwischen dem implantierbaren medizinischen Gerät und dem externen Gerät bilden.

Das implantierbare medizinische Gerät 10 ist im Ausführungsbeispiel als implantierbarer Herzschrittmacher mit einem Gehäuse 12 und einem Header 14 dargestellt. Neben den üblichen Komponenten eines derartigen implantierbaren medizinischen Gerätes besitzt das implantierbare medizinische Gerät 10 eine magnetische Schnittstelle 16 und eine Funkschnittstelle 18, die beide mit einer Steuereinheit 20 des implantierbaren medizinischen Gerätes 10 verbunden sind. Die Funkschnittstelle 18 ist mit einer Antenne 22 verbunden, die außerhalb des metallischen Gehäuses 12 des implantierbaren medizinischen Gerätes 10 im Header 14 untergebracht ist. Dieser besteht aus Kunststoff und schirmt daher elektrische Wechselfelder nicht ab.

Eine Empfängerspule 24 der magnetischen Schnittstelle 16 ist in dem metallischen Gehäuse 12 des implantierbaren medizinischen Gerätes 10 untergebracht. Über die Empfängerspule 24 kann die magnetische Schnittstelle Steuersequenzen empfangen, die mittels eines magnetischen Wechselfeldes übertragen werden. Das magnetische Wechselfeld ist in der Lage, das metallische Gehäuse 12 des implantierbaren medizinischen Gerätes zu durchdringen.

Das externe Gerät 30 besitzt ebenfalls eine Funkschnittstelle 32 mit einer Antenne 34 sowie eine induktive Sendeeinheit 36 mit einer Induktionsspule 38.

Die induktive Sendeeinheit 36 ist dazu ausgebildet, über die Induktionsspule 38 Steuersequenzen mittels eines magnetischen Wechselfeldes auszusenden.

Die Funkschnittstelle 32 des externen Gerätes 30 ist dazu ausgebildet, bidirektional mit der Funkschnittstelle 18 des implantierbaren medizinischen Gerätes zu kommunizieren.

Wie in Fig. 1 angedeutet ist, ist wenigstens die Induktionsspule 38 der induktiven Sendeeinheit 36 in einem vergleichsweise beweglichen, über Draht mit dem Rest des externen Gerätes 30 verbundenen Bestandteil des externen Gerätes 30 untergebracht, der als Programmierkopf dient. Insgesamt dient das externe Gerät 30 als Programmiergerät für das implantierbare medizinische Gerät 10. Durch die Unterbringung der Induktionsspule 38 der induktiven Sendeeinheit 36 in dem beweglichen Programmierkopf 40 des externen Programmiergerätes 30 kann die Induktionsspule 38 leicht in die Nähe des implantierbaren medizinischen Gerätes gebracht werden, auch wenn dies im Körper eines Patienten implantiert sein sollte.

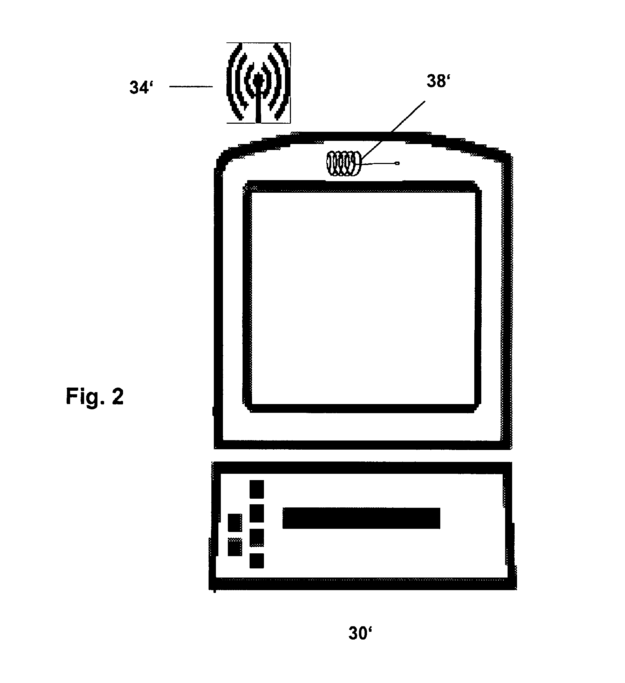

Alternativ kann die Induktionsspule 38' der induktiven Sendeeinheit 36 auch im Gehäuse des externen Gerätes 30' untergebracht sein, wie dies Fig. 2 darstellt. Das externe Gerät 30 kann dabei selbst so ausgebildet sein, dass es nach Art eines mobilen Telefons in der Hand zu halten ist.

Die Funkschnittstellen 18 und 32 des implantierbaren medizinischen Gerätes 10 bzw. des externen Gerätes 30 sind dazu ausgebildet, Daten, beispielsweise vom implantierbaren medizinischen Gerät 10 gesammelte physiologische Daten oder Programmierdaten, per Funk mittels eines elektrischen Wechselfeldes drahtlos zu übertragen, und zwar vorzugsweise in dem speziell für diese Zwecke vorgesehenen MICS-Frequenzband.

Außerdem ist ein drahtloser Austausch von Steuersequenzen zwischen dem externen Gerät 30 und dem implantierbaren medizinischen Gerät 10 über die induktive Sendeeinheit 36 und die magnetische Schnittstelle 18 möglich. Wie den Figuren 1 und 2 zu entnehmen ist, kann dazu das externe Gerät 30 eine oder mehrere Induktionsspulen an verschiedenen Orten aufweisen.

Die Funkschnittstelle 18 des implantierbaren medizinischen Gerätes 10 ist so ausgebildet, dass sie sowohl einen Einschalt- als auch einen Ausschaltzustand der eingangs beschriebenen Art annehmen kann. Um eine in Fig. 1 nicht abgebildete Batterie des implantierbaren medizinischen Gerätes 10 möglichst zu schonen, ist es wünschenswert, wenn die Funkschnittstelle 18 des implantierbaren Gerätes 10 nur dann aktiviert wird, wenn sie zur drahtlosen Datenübertragung zwischen implantierbarem medizinischen Gerät 10 und externem Gerät 30 tatsächlich gebraucht wird. Die Funkschnittstelle 18 des implantierbaren medizinischen Gerätes 10 ist daher so ausgebildet, dass sie sich nach erfolgreich abgeschlossener Datenübertragung selbständig ausschaltet, d.h. in ihren Ausschaltzustand versetzt.

Um die Funkschnittstelle 18 des implantierbaren medizinischen Gerätes 10 auch von außerhalb des implantierbaren medizinischen Gerätes 10 aktivieren, d.h. in den Einschaltzustand versetzen zu können, ist die Funkschnittstelle 18 wenigstens indirekt (im Ausführungsbeispiel über die Steuereinheit 20) mit einer magnetischen Schnittstelle 16 verbunden. Die magnetische Schnittstelle 16 ist dazu ausgebildet, über ihre Empfängerspule 24 Steuersequenzen zu empfangen, die ein Datenübertragungs-Startsignal repräsentieren. Die Steuersequenzen sind durch ein magnetisches Wechselfeld repräsentiert, welches von der magnetischen Schnittstelle 16 demoduliert wird. Anschließend können die Steuersequenzen durch die magnetische Schnittstelle 16 oder die Steuereinheit 20 decodiert werden. Stellen die empfangenen Steuersequenzen ein Datenübertragungs-Startsignal dar, generiert die Steuereinheit 20 ein Funkschnittstellen-Einschaltsignal, welches bewirkt, dass die Funkschnittstelle 18 des implantierbaren medizinischen Gerätes 10 aktiviert oder eingeschaltet, d.h. in ihren Einschaltzustand versetzt, wird.

Wie zuvor bereits beschrieben kann das implantierbare medizinische Gerät 10 auch so ausgebildet sein, dass nicht jedes beliebige Datenübertragungs-Startsignal sofort zum Erzeugen eines Funkschnittstellen-Einschaltsignals führt. Vielmehr kann das jeweilige Datenübertragungs-Startsignal auch eine spezifische Gerätekennung enthalten, welche zur Folge hat, dass nur ein mit dieser Gerätekennung bezeichnetes implantierbares medizinisches Gerät nach Empfangen des Datenübertragungs-Startsignals ein Funkschnittstellen-Einschaltsignal erzeugt. Auch kann das Datenübertragungs-Startsignal eine Funkkanalkennung enthalten, die einen bestimmten Übertragungskanal in dem MICS-Frequenzband kennzeichnet. Dann stellt das implantierbare medizinische Gerät 10 die Funkschnittstelle 18 so ein, dass sie Daten in diesem Übertragungskanal überträgt.

Auch kann das implantierbare medizinische Gerät 10 einen in Fig. 1 nicht dargestellten Zeitgeber enthalten und dazu ausgebildet sein, einer ein Datenübertragungs-Startsignal enthaltende Steuersequenz auch eine Antwortzeit-Kennung zu entnehmen, die einen Zeitpunkt bezeichnet, zu dem das implantierbare medizinische Gerät, insbesondere dessen Steuereinheit 20, ein Funkschnittstellen-Einschaltsignal generiert und damit die Funkschnittstelle 18 einschaltet.

Weitere Details des Ablaufs einer Initiierung einer bidirektionalen Funkdatenübertragung über die Funkschnittstellen 18 und 32 des implantierbaren medizinischen Gerätes 10 bzw. des externen Gerätes 30 ergeben sich aus der nachfolgenden Schilderung alternativer Abläufe.

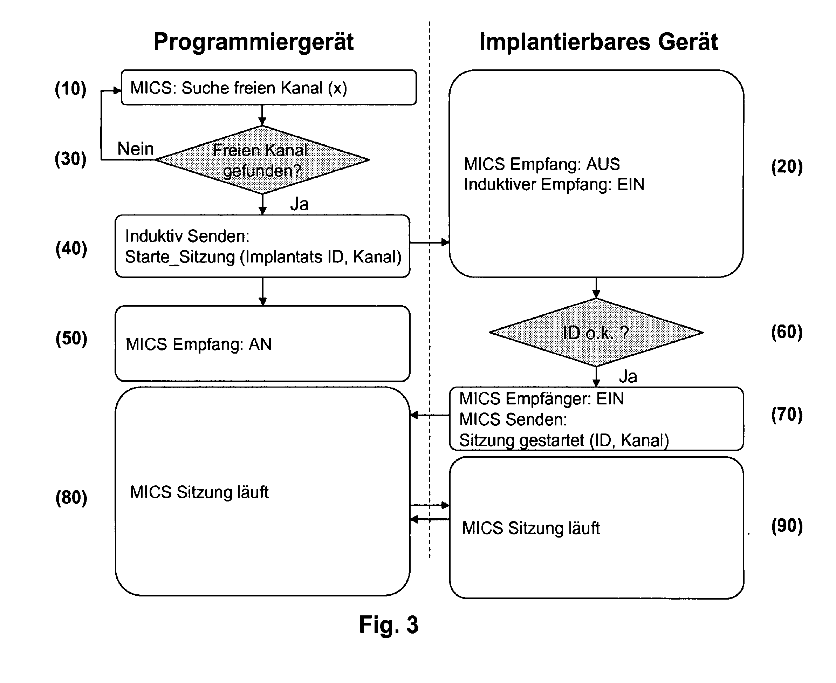

In der folgenden Figur 3 ist der prinzipielle Ablauf der Aktivierung der MICS-Telemetrie eines implantierbaren medizinischen Gerätes über die unidirektionale induktive Telemetrie (d.h. die Datenübertragung über ein magnetisches Wechselfeld) dargestellt:

Zunächst wird durch das Programmiergerät als externes Gerät der im MICS-Band an wenigsten gestörte Kanal ausgewählt (10).

Während dessen ist die MICS-Telemetrie - d.h. die Funkschnittstelle - im implantierbaren medizinischen Geräte abgeschaltet. Der Empfang für die induktive Telemetrie, d.h. die magnetische Schnittstelle des implantierbaren medizinischen Gerätes ist aktiviert (20).

Wurde vom Programmiergerät der am wenigsten gestörte freie Kanal identifiziert (30), sendet das Programmiergerät über die induktive Sendeeinheit ein Datenübertragungsstartsignal zur Aktivierung der MICS-Funkschnittstelle an das implantierbare medizinische Gerät. Dieses Datenübertragungsstartsignal enthält die Information über die zu aktivierende Implantat-ID in Form einer Gerätekennung und die MICS-Kanalinformation für den Verbindungsaufbau (40) in Form einer Funkkanalkennung.

Das Programmiergerät startet unmittelbar nach Senden des Datenübertragungsstartsignals seine eigene MICS-Funkschnittstelle und wartet auf die Bestätigung der Aktivierung des implantierbaren medizinischen Gerätes (50).

Das implantierbare medizinische Gerät empfängt über den induktiven Empfänger seiner magnetischen Schnittstelle dieses Datenübertragungsstartsignal und vergleicht die gesendete Gerätekennung (Implantat-ID) mit der eigenen Implantat-ID. Sofern diese übereinstimmen (60), wird im implantierbaren medizinischen Gerät die MICS-Funkschnittstelle aktiviert und auf dem übermittelten MICS-Kanal eine Bestätigung der Aktivierung sowie der Implantat ID in Form eines Funkübertragungs-Startsignals an das Programmiergerät gesendet (70).

Anschließend wird die bidirektionale Datenübertragung auf dem MICS-Band zwischen Programmiergerät (80) und implantierbaren medizinischen Gerät (90) fortgesetzt.

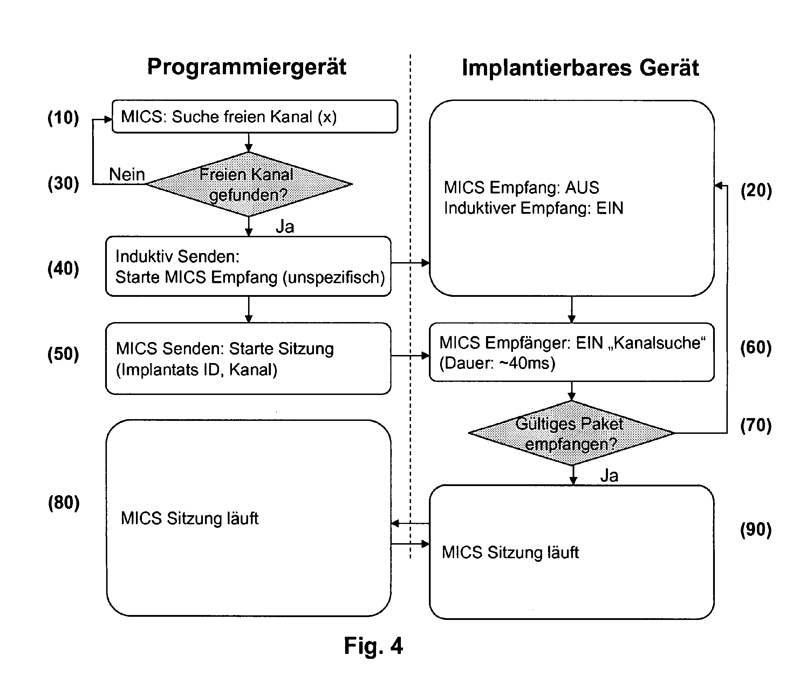

In der Figur 4 ist eine alternative Implementierung dargestellt. Hier wird davon ausgegangen, dass über die induktive Telemetrie nur eine sehr unspezifische Information über eine große Reichweite übertragen werden kann.

Das Programmiergerät als externes Gerät sucht zunächst den freien und am wenigsten gestörten Kanal im MICD-Band (10). Im implantierbaren medizinischen Gerät ist während dessen der Empfänger der magnetischen Schnittstelle für die induktive Telemetrie aktiviert, der (stromintensive) MICS-Empfang über die Funkschnittstelle jedoch abgeschaltet (20).

Wurde vom Programmiergerät ein freier Kanal gefunden (30), so sendet dieser ein unspezifisches Kommando als Datenübertragungsstartsignal auf dem induktiven Weg (40). Dieses Kommando führt bei Empfang durch das implantierbare medizinische Gerät zur kurzzeitigen Aktivierung des MICS-Empfängers der Funkschnittstelle, die dann in einem definierten Zeitfenster nach Aktivierung eine Kanalsuche ausführt (60). Empfängt die Funkschnittstelle des implantierbaren medizinischen Gerätes ein gültiges Datenpaket auf einem der MICS-Kanäle, starten das implantierbare medizinische Gerät und das Programmiergerät eine Kommunikationssitzung auf dem MICS-Protokoll (80, 90). Empfängt die Funkschnittstelle des implantierbaren medizinischen Gerätes kein gültiges Paket, dann deaktiviert das implantierbare medizinische Gerät selbständig seine Funkschnittstelle und kehrt in den Ausgangszustand (20) zurück.

Die erfindungsgemäße Lösung bietet den Vorteil, dass eine "Fernaktivierung" der Funkschnittstelle eines implantierbaren medizinischen Gerätes ohne den zusätzlichen Aufwand eines separaten RF-Aktivators oder eines zusätzlichen RF-Empfängers möglich wird. Des Weiteren ist das alternativ mögliche initiale Auflegen des Programmierkopfes im Nahbereich des implantierbaren medizinischen Gerätes nicht erforderlich, so dass eine RF-Aktivierung im Operationssaal möglich ist, ohne in den sterilen Bereich mit dem Programmierkopf eindringen zu müssen. Ebenso ist eine Suchfunktion alle in Reichweite befindlichen implantierbaren medizinischen Geräte realisierbar (Ambulanz, Wartezimmer, Rettungsstelle).

## Patentansprüche

1. Implantierbares medizinisches Gerät (10) mit einer Steuereinheit (20), die mit einer bidirektionalen Funkschnittstelle (18) und einer magnetischen Schnittstelle (16) verbunden ist,
von denen die bidirektionale Funkschnittstelle (18) zur bidirektionalen drahtlosen Datenübertragung mittels eines elektrischen Wechselfeldes zwischen dem medizinischen Gerät und einem externen Gerät ausgebildet ist und die wenigstens einen Abschaltzustand und einen Einschaltzustand annehmen kann, wobei eine drahtlose Datenübertragung nur im Einschaltzustand möglich ist und die Funkschnittstelle im Abschaltzustand keine oder fast keine Energie benötigt,
und von denen die magnetische Schnittstelle (16) ausgebildet ist permanent mittels eines magnetischen Wechselfeldes übertragene Steuersignale seitens eines externen Gerätes empfangen zu können,
und von denen die magnetische Schnittstelle (16) weiter ausgebildet ist, ein eine Funkkanalkennung enthaltendes Datenübertragungsstartsignal zu empfangen und derart zu verarbeiten, dass die magnetische Schnittstelle oder die Steuereinheit in Reaktion auf den Empfang dieses Datenübertragungsstartsignals ein Funkschnittstelleneinschaltsignal erzeugt
und die bidirektionale Funkschnittstelle (18) wenigstens mittelbar mit der magnetischen Schnittstelle verbunden und ausgebildet ist, auf ein Funkschnittstelleneinschaltsignal hin von dem Abschaltzustand in den Einschaltzustand zu wechseln und anschließend die Funkschnittstelle zur Datenübertragung in einem durch die Funkkanalkennung identifizierten Übertragungskanal einzurichten und den Empfang eines Datenpaketes über die Funkschnittstelle abzuwarten, bevor das implantierbare medizinische Gerät über die Funkschnittstelle selbst ein Datenpaket aussendet.

2. Implantierbares medizinisches Gerät nach Anspruch 1, **dadurch gekennzeichnet, dass** dessen magnetische Schnittstelle (16) oder dessen Steuereinheit (20) dazu ausgebildet ist, nach Empfang eines Datenübertragungs-Startsignals nur dann ein Funkschnittstellen-Einschaltsignal zu erzeugen, wenn das über die magnetische Schnittstelle empfangene Datenübertragungs-Startsignal eine für das spezielle implantierbare medizinische Gerät spezifische Geräte-Kennung enthält.

3. Implantierbares medizinisches Gerät nach einem der Ansprüche 1 bis 2, **dadurch gekennzeichnet, dass** das implantierbare medizinische Gerät derart ausgestaltet ist, dass die Aktivierbarkeit der Funkschnittstelle durch ein Datenübertragungsstartsignal mittels eines von der magnetischen Schnittstelle zu empfangenden Steuerkommandos zu aktivieren bzw. zu deaktivieren ist.

4. Implantierbares medizinisches Gerät nach einem der Ansprüche 2 bis 3, **dadurch gekennzeichnet, dass** das implantierbare medizinische Geräte dazu ausgebildet ist, einem Datenübertragungsstartsignal neben einer Gerätekennung eine Antwortzeitpunktskennung zu entnehmen und das Funkschnittstelleneinschaltsignal erst zu generieren, wenn ein durch die Antwortzeitpunktskennung definierter Antwortzeitpunkt erreicht ist

5. Externes Gerät mit einer bidirektionalen Funkschnittstelle für die Übertragung von Daten mittels eines hochfrequenten elektromagnetischen Wechselfeldes, mit einer induktiven Sendeeinheit zum Übertragen eines Datenübertragungs-Startsignals an ein implantierbares medizinisches Gerät mittels eines magnetischen Wechselfeldes, die ausgebildet ist, ein Datenübertragungsstartsignal zu generieren, welches eine Funkkanalkennung enthält, welche einen für die bidirektionale Datenübertragung über die Funkschnittstelle zu verwendenden Übertragungskanal (Frequenzbereich) identifiziert,
**dadurch gekennzeichnet, dass** das externe Gerät ausgebildet ist, vor dem Generieren des Datenübertragungs-Startsignals, zunächst die zur Verfügung stehenden Übertragungskanäle zu überprüfen, um einen freien und möglichst ungestörten Übertragungskanal zu ermitteln und anschließend dem auszusendenden Datenübertragungs-Startsignal die Funkkanalkennung desjenigen Übertragungskanals hinzuzufügen, der frei und am wenigsten gestört ist.

6. Externes Gerät nach Anspruch 5, **dadurch gekennzeichnet, dass** das externe Gerät ausgebildet ist, ein Datenübertragungsstartsignal für die Übertragung über die induktive Senseeinheit zu generieren, welches eine Gerätekennung enthält, die ein implantierbares medizinisches Gerät eindeutig kennzeichnet.

7. Externes Gerät nach einem der Ansprüche 5 bis 6, **dadurch gekennzeichnet, dass** das externe Gerät dazu ausgebildet ist, ein Datenübertragungs-Startsignal zu generieren, welches eine Antwortzeitkennung enthält, die einen Zeitpunkt bestimmt, zu welchem ein implantierbares medizinisches Gerät seine Funkübertragungsschnittstelle aktiviert.

8. System zur bidirektionalen Datenübertragung zwischen einem implantierbaren medizinischen Gerät und einem externen Gerät, **gekennzeichnet durch** ein implantierbares medizinisches Gerät nach einem der Ansprüche 1 bis 4 und ein externes Gerät nach einem der Ansprüche 5 bis 7.

## Claims

1. An implantable medical device (10), comprising a control unit (20) that is connected to a bidirectional radio interface (18) and a magnetic interface (16),
of which the bidirectional radio interface (18) is configured for bidirectional wireless data transmission by means of an electric alternating field between the medical device and an external device and can adopt at least one power-off state and one power-on state, wherein a wireless data transmission is possible only in the power-on state and the radio interface requires no or hardly any energy in the power-off state,
and of which the magnetic interface (16) is configured to be able to permanently receive control signals transmitted by means of a magnetic alternating field from an external device,
and of which the magnetic interface (16) is also configured to receive a data transmission start signal containing a radio channel identifier and to process said signal in such a way that the magnetic interface or the control unit generates a radio interface power-on signal in response to the receipt of this data transmission start signal,
and the bidirectional radio interface (18) is connected at least indirectly to the magnetic interface and is configured, upon receipt of a radio interface power-on signal, to change from the power-off state into the power-on state and then to set up the radio interface for data transmission in a transmission channel identified by the radio channel identifier and to await the receipt of a data packet via the radio interface before the implantable medical device itself sends a data packet via the radio interface.

2. The implantable medical device according to Claim 1, **characterised in that** the magnetic interface (16) thereof or the control unit (20) thereof is configured, following receipt of a data transmission start signal, to only then generate a radio interface power-on signal if the data transmission start signal received via the magnetic interface contains a device identifier specific for the special implantable medical device.

3. The implantable medical device according to one of Claims 1 to 2, **characterised in that** the implantable medical device is configured in such a way that the activatability of the radio interface by a data transmission start signal is to be activated and deactivated by means of a control command to be received by the magnetic interface.

4. The implantable medical device according to one of Claims 2 to 3, **characterised in that** the implantable medical device, besides a device identifier, is configured to also infer a response time identifier from a data transmission start signal and to generate the radio interface power-on signal only when a response time defined by the response time identifier is reached.

5. An external device comprising a bidirectional radio interface for the transmission of data by means of a high-frequency electromagnetic alternating field and comprising an inductive transmitter unit for transmitting a data transmission start signal to an implantable medical device by means of a magnetic alternating field, said inductive transmitter unit being configured to generate a data transmission start signal containing a radio channel identifier which identifies a transmission channel (frequency range) to be used for the bidirectional data transmission via the radio interface,
**characterised in that** the external device is configured to first check the available transmission channels prior to the generation of the data transmission start signal in order to determine a free transmission channel free from interference where possible and then to add to the data transmission start signal to be transmitted the radio channel identifier of that transmission channel which is free and subject to least interference.

6. The external device according to Claim 5, **characterised in that** the external device is configured to generate a data transmission start signal for the transmission via the inductive transmitter unit, said signal containing a device identifier that clearly identifies an implantable medical device.

7. The external device according to one of Claims 5 to 6, **characterised in that** the external device is configured to generate a data transmission start signal that contains a response time identifier which determines a time at which an implantable medical device activates its radio transmission interface.

8. A system for bidirectional data transmission between an implantable medical device and an external device, **characterised by** an implantable medical device according to one of Claims 1 to 4 and an external device according to one of Claims 5 to 7.

## Revendications

1. Dispositif médical implantable (10) avec une unité de commande (20) reliée à une interface radio bidirectionnelle (18) et à une interface magnétique (16),
parmi lesquelles l'interface radio bidirectionnelle (18) est conçue pour une transmission de données au moyen d'un champ alternatif électrique entre le dispositif médical et un dispositif externe, et peut se mettre dans au moins un état éteint et un état allumé, une transmission de données sans fil n'étant possible que dans l'état allumé, et l'interface radio ne nécessitant aucune ou quasiment aucune énergie dans l'état éteint,
et parmi lesquelles l'interface magnétique (16) est conçue pour pouvoir recevoir en permanence des signaux de commande transmis au moyen d'un champ magnétique alternatif, venant d'un dispositif externe,
et parmi lesquelles l'interface magnétique (16) est en outre conçue pour recevoir un signal de départ de transmission de données contenant un identificateur de canal radio, et pour traiter celui-ci de manière à ce que l'interface magnétique ou l'unité de commande produise un signal d'allumage d'interface radio en réaction à la réception de ce signal de départ de transmission de données,
et l'interface radio bidirectionnelle (18) étant au moins indirectement reliée à l'interface magnétique et conçue pour passer de l'état éteint à l'état allumé en réponse à un signal d'allumage d'interface radio, et pour ensuite configurer l'interface radio pour la transmission de données dans un canal de transmission identifié par l'identificateur radio, et pour attendre la réception d'un paquet de données par le biais de l'interface radio, avant que le dispositif médical implantable n'envoie lui-même un paquet de données par le biais de l'interface radio.

2. Dispositif médical implantable selon la revendication 1, **caractérisé en ce que** l'interface magnétique (16) ou l'unité de commande (20) de celui-ci est conçue pour produire un signal d'allumage d'interface radio, après réception d'un signal de départ de transmission de données, uniquement lorsque le signal de départ de transmission de données reçu par le biais de l'interface magnétique contient un identificateur de dispositif spécifique au dispositif médical implantable.

3. Dispositif médical implantable selon l'une des revendications 1 à 2, **caractérisé en ce que** le dispositif médical implantable est conçu de manière à ce que la capacité d'activation de l'interface radio puisse être activée ou désactivée par un signal de départ de transmission de données au moyen d'une instruction de commande à recevoir de l'interface magnétique.

4. Dispositif médical implantable selon l'une des revendications 2 à 3, **caractérisé en ce que** le dispositif médical implantable est conçu pour prélever un identificateur de moment de réponse en plus d'un identificateur de dispositif à partir d'un signal de départ de transmission de données, et pour générer le signal d'allumage d'interface radio d'interface radio seulement lorsqu'un moment de réponse défini par l'identificateur de moment de réponse.

5. Dispositif externe avec une interface radio bidirectionnelle pour la transmission de données au moyen d'un champ électromagnétique alternatif à haute fréquence, avec une unité d'émission inductive pour la transmission d'un signal de départ de transmission de données à un dispositif médical implantable au moyen d'un champ magnétique alternatif, laquelle est conçue pour générer un signal de départ de transmission de données contenant un identificateur de canal radio identifiant un canal de transmission (domaine de fréquence) à utiliser pour la transmission de données bidirectionnelle par le biais de l'interface radio,
**caractérisé en ce que** le dispositif externe est conçu pour vérifier tout d'abord les canaux de transmission disponibles, avant de générer le signal de départ de transmission de données, afin de déterminer un canal de transmission libre et peu parasité, et pour ensuite associer l'identificateur de canal radio du canal de transmission libre et le moins parasité au signal de départ de transmission de données.

6. Dispositif externe selon la revendication 5, **caractérisé en ce que** le dispositif externe est conçu pour générer un signal de départ de transmission de données pour la transmission par le biais de l'unité de détection inductive, lequel contient un identificateur de dispositif désignant un dispositif médical implantable de façon univoque.

7. Dispositif externe selon l'une des revendications 5 à 6, **caractérisé en ce que** le dispositif externe est conçu pour générer un signal de départ de transmission de données contenant un identificateur de temps de réponse déterminant un moment dans lequel un dispositif médical implantable active son interface de transmission radio.

8. Système pour la transmission de données bidirectionnelle entre un dispositif médical implantable et un dispositif externe, **caractérisé par** un dispositif médical implantable selon l'une des revendications 1 à 4 et un dispositif externe selon l'une des revendications 5 à 7.
